# EUROPEAN PATENT APPLICATION

(11) **EP 3 889 269 A2**
(43) Date of publication of application: **06.10.2021**
(21) Application number: 19888557.6
(22) Date of filing: 26.11.2019
(51) Int. Cl.: C12Q 1/68, C12N 15/11

(54) **DNA-CUTTING AGENT**

(30) Priority: 26.11.2018 RU 2018141524
(71) Applicant: Joint Stock Company "Biocad", St.Petersburg 198515 (RU)
(72) Inventor: SEVERINOV, Konstantin Viktorovich, Moscow, 119333 (RU); SHMAKOV, Sergey Anatolevich, Voskresensk, 140200 (RU); ARTAMONOVA, Daria Nikolaevna, Moscow, 121359 (RU); GORYANIN, Ignatiy Igorevich, Moscow, 117587 (RU); MUSHAROVA, Olga Sergeevna, Moscow, 123098 (RU); PISKUNOVA, Iuliia Valerevna, Moscow, 142784 (RU); FEDOROVA, Iana Vitalevna, Gatchina, 188301 (RU); ZYUBKO, Tatyana Igorevna, St.Petersburg, 197374 (RU); KHODORKOVSKIY, Mikhail Alekseevich, St.Petersburg, 195426 (RU); POBEGALOV, Georgii Evgenevich, St. Petersburg, 198205 (RU); ARSENIEV, Anatolii Nikolaevich, St. Petersburg, 195112 (RU); SELKOVA, Polina Anatolevna, Votkinsk, 427439 (RU); VASILEVA, Aleksandra Andreevna, St. Petersburg, 198334 (RU); ARTAMONOVA, Tatiana Olegovna, St.Petersburg, 199406 (RU); ABRAMOVA, Marina Viktorovna, St.Petersburg, 194021 (RU)
(74) Representative: Held, Stephan
(86) International application number: PCT/RU2019/050229
(87) International publication number: WO 2020/111983

(57) **Abstract**

The present invention describes a novel bacterial nuclease of the CRISPR-Cas9 system from the bacterium Clostridium celluloliticum, as well as the use thereof to form strictly specific double-strand breaks in a DNA molecule. This nuclease has unusual properties and may be used as a tool for introducing modifications at strictly defined sites in the genomic DNA sequence of unicellular or multicellular organisms. Thus, the versatility of the available CRISPR-Cas9 systems is increased, which will enable the use of Cas9 nucleases from various organisms for cutting genomic or plasmid DNA in a larger number of specific sites and in wider temperature ranges. Further, provided is more facile editing of the genome of the biotechnologically significant bacterium Clostridium celluloliticum.

sequence listing

<110> JSC "BIOCAD"

<120> DNA-CUTTING AGENT

<130> 417252

<140> PCT/RU2019/050229
<141> 2019-11-26

<150> RU 2018141524
<151> 2018-11-26

<160> 2

<170> BiSSAP 1.3.6

<210> 1
<211> 1021
<212> PRT
<213> Clostridium cellulolyticum

<400> 1
Met Lys Tyr Thr Leu Gly Leu Asp Val Gly Ile Ala Ser Val Gly Trp
1               5                   10                  15
Ala Val Ile Asp Lys Asp Asn Asn Lys Ile Ile Asp Leu Gly Val Arg
            20                  25                  30
Cys Phe Asp Lys Ala Glu Glu Ser Lys Thr Gly Glu Ser Leu Ala Thr
        35                  40                  45
Ala Arg Arg Ile Ala Arg Gly Met Arg Arg Arg Ile Ser Arg Arg Ser
    50                  55                  60
Gln Arg Leu Arg Leu Val Lys Lys Leu Phe Val Gln Tyr Glu Ile Ile
65                  70                  75                  80
Lys Asp Ser Ser Glu Phe Asn Arg Ile Phe Asp Thr Ser Arg Asp Gly
                85                  90                  95
Trp Lys Asp Pro Trp Glu Leu Arg Tyr Asn Ala Leu Ser Arg Ile Leu
            100                 105                 110
Lys Pro Tyr Glu Leu Val Gln Val Leu Thr His Ile Thr Lys Arg Arg
        115                 120                 125
Gly Phe Lys Ser Asn Arg Lys Glu Asp Leu Ser Thr Thr Lys Glu Gly
    130                 135                 140
Val Val Ile Thr Ser Ile Lys Asn Asn Ser Glu Met Leu Arg Thr Lys
145                 150                 155                 160
Asn Tyr Arg Thr Ile Gly Glu Met Ile Phe Met Glu Thr Pro Glu Asn
                165                 170                 175
Ser Asn Lys Arg Asn Lys Val Asp Glu Tyr Ile His Thr Ile Ala Arg
            180                 185                 190
Glu Asp Leu Leu Asn Glu Ile Lys Tyr Ile Phe Ser Ile Gln Arg Lys
        195                 200                 205
Leu Gly Ser Pro Phe Val Thr Glu Lys Leu Glu His Asp Phe Leu Asn
    210                 215                 220
Ile Trp Glu Phe Gln Arg Pro Phe Ala Ser Gly Asp Ser Ile Leu Ser
225                 230                 235                 240
Lys Val Gly Lys Cys Thr Leu Leu Lys Glu Glu Leu Arg Ala Pro Thr
                245                 250                 255
Ser Cys Tyr Thr Ser Glu Tyr Phe Gly Leu Leu Gln Ser Ile Asn Asn

            260                 265                 270
Leu Val Leu Val Glu Asp Asn Asn Thr Leu Thr Leu Asn Asn Asp Gln
        275                 280                 285
Arg Ala Lys Ile Ile Glu Tyr Ala His Phe Lys Asn Glu Ile Lys Tyr
    290                 295                 300
Ser Glu Ile Arg Lys Leu Leu Asp Ile Glu Pro Glu Ile Leu Phe Lys
305                 310                 315                 320
Ala His Asn Leu Thr His Lys Asn Pro Ser Gly Asn Asn Glu Ser Lys
                325                 330                 335
Lys Phe Tyr Glu Met Lys Ser Tyr His Lys Leu Lys Ser Thr Leu Pro
            340                 345                 350
Thr Asp Ile Trp Gly Lys Leu His Ser Asn Lys Glu Ser Leu Asp Asn
        355                 360                 365
Leu Phe Tyr Cys Leu Thr Val Tyr Lys Asn Asp Asn Glu Ile Lys Asp
    370                 375                 380
Tyr Leu Gln Ala Asn Asn Leu Asp Tyr Leu Ile Glu Tyr Ile Ala Lys
385                 390                 395                 400
Leu Pro Thr Phe Asn Lys Phe Lys His Leu Ser Leu Val Ala Met Lys
                405                 410                 415
Arg Ile Ile Pro Phe Met Glu Lys Gly Tyr Lys Tyr Ser Asp Ala Cys
            420                 425                 430
Asn Met Ala Glu Leu Asp Phe Thr Gly Ser Ser Lys Leu Glu Lys Cys
        435                 440                 445
Asn Lys Leu Thr Val Glu Pro Ile Ile Glu Asn Val Thr Asn Pro Val
    450                 455                 460
Val Ile Arg Ala Leu Thr Gln Ala Arg Lys Val Ile Asn Ala Ile Ile
465                 470                 475                 480
Gln Lys Tyr Gly Leu Pro Tyr Met Val Asn Ile Glu Leu Ala Arg Glu
                485                 490                 495
Ala Gly Met Thr Arg Gln Asp Arg Asp Asn Leu Lys Lys Glu His Glu
            500                 505                 510
Asn Asn Arg Lys Ala Arg Glu Lys Ile Ser Asp Leu Ile Arg Gln Asn
        515                 520                 525
Gly Arg Val Ala Ser Gly Leu Asp Ile Leu Lys Trp Arg Leu Trp Glu
    530                 535                 540
Asp Gln Gly Gly Arg Cys Ala Tyr Ser Gly Lys Pro Ile Pro Val Cys
545                 550                 555                 560
Asp Leu Leu Asn Asp Ser Leu Thr Gln Ile Asp His Ile Tyr Pro Tyr
                565                 570                 575
Ser Arg Ser Met Asp Asp Ser Tyr Met Asn Lys Val Leu Val Leu Thr
            580                 585                 590
Asp Glu Asn Gln Asn Lys Arg Ser Tyr Thr Pro Tyr Glu Val Trp Gly
        595                 600                 605
Ser Thr Glu Lys Trp Glu Asp Phe Glu Ala Arg Ile Tyr Ser Met His
    610                 615                 620
Leu Pro Gln Ser Lys Glu Lys Arg Leu Leu Asn Arg Asn Phe Ile Thr
625                 630                 635                 640
Lys Asp Leu Asp Ser Phe Ile Ser Arg Asn Leu Asn Asp Thr Arg Tyr
                645                 650                 655
Ile Ser Arg Phe Leu Lys Asn Tyr Ile Glu Ser Tyr Leu Gln Phe Ser
            660                 665                 670
Asn Asp Ser Pro Lys Ser Cys Val Val Cys Val Asn Gly Gln Cys Thr
        675                 680                 685
Ala Gln Leu Arg Ser Arg Trp Gly Leu Asn Lys Asn Arg Glu Glu Ser
    690                 695                 700
Asp Leu His His Ala Leu Asp Ala Ala Val Ile Ala Cys Ala Asp Arg
705                 710                 715                 720
Lys Ile Ile Lys Glu Ile Thr Asn Tyr Tyr Asn Glu Arg Glu Asn His

725                 730                 735
Asn Tyr Lys Val Lys Tyr Pro Leu Pro Trp His Ser Phe Arg Gln Asp
            740                 745                 750
Leu Met Glu Thr Leu Ala Gly Val Phe Ile Ser Arg Ala Pro Arg Arg
        755                 760                 765
Lys Ile Thr Gly Pro Ala His Asp Glu Thr Ile Arg Ser Pro Lys His
    770                 775                 780
Phe Asn Lys Gly Leu Thr Ser Val Lys Ile Pro Leu Thr Thr Val Thr
785                 790                 795                 800
Leu Glu Lys Leu Glu Thr Met Val Lys Asn Thr Lys Gly Gly Ile Ser
                805                 810                 815
Asp Lys Ala Val Tyr Asn Val Leu Lys Asn Arg Leu Ile Glu His Asn
            820                 825                 830
Asn Lys Pro Leu Lys Ala Phe Ala Glu Lys Ile Tyr Lys Pro Leu Lys
        835                 840                 845
Asn Gly Thr Asn Gly Ala Ile Ile Arg Ser Ile Arg Val Glu Thr Pro
    850                 855                 860
Ser Tyr Thr Gly Val Phe Arg Asn Glu Gly Lys Gly Ile Ser Asp Asn
865                 870                 875                 880
Ser Leu Met Val Arg Val Asp Val Phe Lys Lys Lys Asp Lys Tyr Tyr
                885                 890                 895
Leu Val Pro Ile Tyr Val Ala His Met Ile Lys Lys Glu Leu Pro Ser
            900                 905                 910
Lys Ala Ile Val Pro Leu Lys Pro Glu Ser Gln Trp Glu Leu Ile Asp
        915                 920                 925
Ser Thr His Glu Phe Leu Phe Ser Leu Tyr Gln Asn Asp Tyr Leu Val
    930                 935                 940
Ile Lys Thr Lys Lys Gly Ile Thr Glu Gly Tyr Tyr Arg Ser Cys His
945                 950                 955                 960
Arg Gly Thr Gly Ser Leu Ser Leu Met Pro His Phe Ala Asn Asn Lys
                965                 970                 975
Asn Val Lys Ile Asp Ile Gly Val Arg Thr Ala Ile Ser Ile Glu Lys
            980                 985                 990
Tyr Asn Val Asp Ile Leu Gly Asn Lys Ser Ile Val Lys Gly Glu Pro
        995                 1000                1005
Arg Arg Gly Met Glu Lys Tyr Asn Ser Phe Lys Ser Asn
    1010                1015                1020

<210> 2
<211> 3066
<212> DNA
<213> Clostridium cellulolyticum

<400> 2
atgaaatata cattaggtct tgatgttgga attgcttctg taggttgggc ggtaattgat      60

aaggataata ataaaattat tgacttagga gttagatgtt ttgataaagc agaggaatct     120

aaaacgggtg agtcacttgc aacagctaga aggatagcta gaggtatgag aagaagaatt     180

tcgagaagat cccaaaggct acgtttagtt aaaaaactgt ttgttcaata tgaaattatt     240

aaagattcaa gtgaatttaa ccggatattt gacacttcgc gggatgggtg gaaagatcct     300

tgggaattaa ggtataatgc tttatcaaga atacttaaac cttatgaact tgttcaggta     360

cttacacata ttactaagag aagaggcttt aaaagtaaca gaaaggaaga cttgtctact     420

acaaaagaag gtgtagttat aactagtatt aaaaacaatt ctgagatgct tcgtacgaaa 480

aattaccgta ctattggaga aatgattttt atggagactc ctgaaaatag taacaaaagg 540

aataaggtag acgagtatat tcacactatt gccagagaag atcttctcaa tgaaataaaa 600

tatatattta gtatacaaag aaagcttgga agcccctttg taactgaaaa attagaacat 660

gactttttga atatatggga atttcaacgt ccttttgcca gtggtgatag tatactttca 720

aaagtgggaa agtgtaccct gctaaaggag gagttgagag caccgacttc ctgttacaca 780

tcagaatatt ttggattact tcaatcgatt aacaatctag ttttggttga agataacaat 840

acattaacat taaataatga tcaaagagca aaaataatag aatatgctca tttcaagaat 900

gaaatcaagt attctgaaat aagaaaatta ttagatattg aacctgaaat tttattcaaa 960

gcacataatt tgacacacaa aaatccctca ggaaacaatg agagcaaaaa gttttatgaa 1020

atgaagtctt atcataaact gaaaagcaca ttacctacag acatctgggg gaaattgcat 1080

tctaacaagg aatctcttga taatcttttt tactgcctta cggtctataa aaacgataac 1140

gaaataaagg actatttaca agcgaataat cttgattatt taattgaata tatagcaaaa 1200

ttgccaactt tcaacaaatt taaacatcta tctttagttg ccatgaaaag gattattccg 1260

tttatggaaa aagggtataa atatagtgat gcctgtaata tggcggaatt agattttaca 1320

ggttccagca aacttgaaaa gtgtaataag ttaactgttg aaccaattat tgagaatgta 1380

actaatccag ttgtaataag ggctctgacg caagcaagga aagttataaa tgcgattata 1440

cagaagtatg gtctcccgta tatggtaaat atagaacttg cacgtgaagc gggaatgaca 1500

cgtcaggata gagataattt aaaaaaagaa catgaaaaca accgaaaagc aagggaaaaa 1560

atatcagacc taatacgcca aaatggtaga gttgcaagtg gtctggatat actgaaatgg 1620

cgtctttggg aagaccaggg cggaagatgt gcttattccg gcaaaccaat tcctgtttgt 1680

gatttattga atgactcact gactcagata gatcacattt atccgtatag tagaagtatg 1740

gacgattcat atatgaataa agttttagtt ttaaccgacg aaaatcaaaa taaaagaagt 1800

tatacgccat atgaagtatg ggggtcaact gaaaaatggg aggattttga ggcaagaata 1860

tattctatgc atttacctca aagtaaagaa aaaaggcttt tgaacagaaa ctttattaca 1920

aaagatttgg actcatttat ttcaagaaat cttaacgata ctaggtatat atcaaggttt 1980

ttaaaaaact atatagaatc gtatctgcag tttagtaatg attcacctaa gtcttgtgtg 2040

gtctgcgtta atggtcagtg taccgctcag cttagaagta gatgggggtt aaataaaaat 2100

cgagaagagt cagacctgca tcatgccctt gatgcagcag taattgcgtg tgcagataga 2160

aaaataatta aagaaataac aaactattat aatgaaagag aaaaccataa ttataaagtt 2220

aaatatcctt tgccttggca ttcgtttagg caagatttaa tggaaacgtt agcaggtgtt 2280

ttcatatccc gggcacccag aagaaaaatt accgggccag cccatgatga gacaatcaga 2340

tctcccaagc attttaacaa aggtttaact tcggtaaaaa taccgttaac aacagtgacg 2400

ttggaaaaac ttgagacaat ggtaaaaaat acaaaagggg gaatttcaga taaggcagta 2460

tataacgttc taaaaaatag attaatagag cataataata agccattaaa agcttttgct 2520

gaaaaaatat ataaaccact aaaaaatggt acaaacggtg caataattag gagtattcga 2580

gttgagacac catcatatac aggagtattc agaaatgaag gaaaggggat atctgataat 2640

tccttaatgg ttagggttga tgtatttaag aaaaaagata agtattacct tgtgccaata 2700

tatgtagcac atatgataaa aaaagagtta ccttcgaaag ctatagttcc tctgaaacct 2760

gaatctcaat gggagttaat tgatagtact catgaatttc ttttttcact ataccaaaat 2820

gattaccttg ttataaaaac taaaaagggt ataactgagg gctattatag atcttgccac 2880

aggggtaccg gtagcctgag cctaatgcct cattttgcta ataataagaa tgttaaaata 2940

gatattggag ttaggacagc aataagtatt gaaaaatata atgtggatat acttggtaat 3000

aaaagcatag taaaaggaga accaagacgt gggatggaga aatataatag tttcaaatcc 3060

aactaa 3066

## Description

### Field of the Invention

The invention relates to the field of molecular biology and microbiology, in particular, it discloses novel bacterial nucleases of the CRISPR-Cas system. The invention may be used as a tool for strictly specific modification of DNA in various organisms.

### Background of the Invention

Modification of a DNA sequence is one of the topical problems in today's biotechnology field. Editing and modifying the genomes of eukaryotic and prokaryotic organisms, as well as manipulating DNA *in vitro,* require the targeted introduction of double-strand breaks into a DNA sequence. To solve this problem, the following techniques are currently used: artificial nuclease systems containing domains of the zinc finger type, TALEN systems, and bacterial CRISPR-Cas systems. The first two techniques require laborious optimization of a nuclease amino acid sequence for recognition of a specific DNA sequence. In contrast, when it comes to CRISPR-Cas systems, the structures that recognize a DNA target are not proteins, but short guide RNAs. Cutting of a particular DNA target does not require the synthesis of nuclease or its gene *de novo,* but is made by way of using guide RNAs complementary to the target sequence. It makes CRISPR Cas systems convenient and efficient means for cutting various DNA sequences. The technique allows for simultaneous cutting of DNA at several regions using guide RNAs of different sequences. Such an approach is also used to simultaneously modify several genes in eukaryotic organisms.

By their nature, CRISPR-Cas systems are prokaryotic immune systems capable of highly specific introduction of breaks into a viral genetic material (Mojica F. J. M. et al. Intervening sequences of regularly spaced prokaryotic repeats derive from foreign genetic elements //Journal of molecular evolution. -2005. - Vol. 60. - Issue 2. - pp. 174-182). The abbreviation CRISPR-Cas stands for "Clustered Regularly Interspaced Short Palindromic Repeats and CRISPR associated Genes" (Jansen R. et al. Identification of genes that are associated with DNA repeats in prokaryotes //Molecular microbiology. - 2002. - Vol. 43. - Issue 6. - pp. 1565-1575). All CRISPR-Cas systems consist of CRISPR cassettes and genes encoding various Cas proteins (Jansen R. et al., Molecular microbiology. - 2002. - Vol. 43. - Issue 6. - pp. 1565-1575). CRISPR cassettes consist of spacers, each having a unique nucleotide sequence, and repeated palindromic repeats (Jansen R. et al., Molecular microbiology. - 2002. - Vol. 43. - Issue 6. - pp. 1565-1575). The transcription of CRISPR cassettes followed by processing thereof results in the formation of guide crRNAs, which together with Cas proteins form an effector complex (Brouns S. J. J. et al. Small CRISPR RNAs guide antiviral defense in prokaryotes / / Science. - 2008. - Vol. 321. - Issue 5891. - pp. 960-964). Due to the complementary pairing between the crRNA and a target DNA site, which is called the protospacer, Cas nuclease recognizes a DNA target and highly specifically introduces a break therein.

CRISPR-Cas systems with a single effector protein are grouped into six different types (types I-VI), depending on Cas proteins that are included in the systems. The type II CRISPR-Cas9 system is characterized in its simple composition and mechanism of activity, i.e. its functioning requires the formation of an effector complex consisting only of one Cas9 protein and two short RNAs as follows: crRNA and tracer RNA (tracrRNA). The tracer RNA complementarily pairs with a crRNA region, originating from CRISPR repeat, to form a secondary structure necessary for the binding of guide RNAs to the Cas effector. The Cas9 effector protein is an RNA-dependent DNA endonuclease with two nuclease domains (HNH and RuvC) that introduce breaks into the complementary strands of target DNA, thus forming a double-strand DNA break (Deltcheva E. et al. CRISPR RNA maturation by trans-encoded small RNA and host factor RNase III //Nature. - 2011. - Vol. 471. - Issue 7340. - p. 602).

Thus far, several CRISPR-Cas nucleases are known that are capable of targeted and specific introduction of double-strand breaks into DNA. One of the main characteristics limiting the use of CRISPR-Cas systems is a PAM sequence that flanks a DNA target from the 3' end and the presence of which is necessary for the correct recognition of DNA by Cas9 nuclease. Various CRISPR-Cas proteins have different PAM sequences that limit the potential for use of the nucleases at any DNA regions. The use of CRISPR-Cas proteins with novel various PAM sequences is necessary to make it possible to modify any DNA region, both *in vitro* and in the genome of living organisms. Modification of eukaryotic genomes also requires the use of the small-sized nucleases to provide AAV-mediated delivery of CRISPR-Cas systems into cells.

Although a number of techniques for cutting DNA and modifying a genomic DNA sequence are known, there is still a need for novel effective means for modifying DNA in various organisms and at strictly specific sites of a DNA sequence. This invention provides a number of properties necessary for solving this problem.

The basis of the invention is the CRISPR Cas system found in the bacteria *Clostridium cellulolyticum.* Anaerobic bacteria *Clostridium cellulolyticum (C. cellulolyticum)* are able to hydrolyze lignocellulose without adding commercial cellulases to form, as end products, lactate, acetate and ethanol (Desvaux M. Clostridium cellulolyticum: model organism of mesophilic cellulolytic clostridia. FEMS Microbiol Rev. 2005 Sep;29(4):741-64). Such an ability of these microorganisms makes them promising candidates for biofuel producers. The use of producer bacteria such as C. *cellulolyticum* in biotechnological production will help to make the raw material processing cycle more efficient, increase efficiency and, ultimately, reduce the load on all components of the biosphere. The genetic engineering methods may significantly improve the microbial metabolic parameters and tilt the balance in favor of the production of more butanol rather than lactate and acetate. For example, a double mutant of the lactate and malate dehydrogenase genes showed the absence of lactate formation and increased butanol yield (Li Y, et al., Combined inactivation of the Clostridium cellulolyticum lactate and malate dehydrogenase genes substantially increases ethanol yield from cellulose and switchgrass fermentations. Biotechnol Biofuels. 2012 Jan 4;5(1):2). Thus far, it has not been possible to develop an effective procedure for producing *C. cellulolyticum* strains with mutations in phosphotransacetylase and acetate kinase genes, which could reduce acetate production. The invention may be used to modify the genome of *Clostridium cellulolyticum,* as well as that of other living organisms.

### Summary of the invention

The object of the present invention is to provide novel means for modifying a genomic DNA sequence of unicellular or multicellular organisms using CRISPR-Cas9 systems. The current systems are of limited use due to a specific PAM sequence that must be present at the 3' end of a DNA region to be modified. Search for novel Cas9 enzymes with other PAM sequences will expand the range of available means for the formation of a double-strand break at desired, strictly specific sites in DNA molecules of various organisms.

To solve this problem, the authors characterized the previously predicted type II CRISPR nuclease CcCas9 for *C. cellulolyticum,* which can be used to introduce directed modifications into the genome of both the above and other organisms. The essential features characterizing the present invention are as follows: (a) short, two-letter PAM sequence, distinct from other known PAM sequences; (b) small size of the characterized CcCas9 protein, which is 1030 amino acid residues (a.a.r.), which is 23 a.a.r. less than that of the known Cas9 enzyme from Staphylococcus aureus (SaCas9); (c) wide operating temperature range of the CcCas9 nuclease, which is active at temperatures from 37 °C to 65 °C with an optimum at 45 ° C, which will make it possible to use same in organisms having various temperatures.

Said problem is solved by using a protein comprising the amino acid sequence of SEQ ID NO: 1, or comprising an amino acid sequence that is at least 95% identical to the amino acid sequence of SEQ ID NO: 1 and differs from SEQ ID NO: 1 only in non-conserved amino acid residues, to form a double-strand break in a DNA molecule, located immediately before the nucleotide sequence 5'-NNNNGNA-3' in said DNA molecule. N is intended to refer to any of the nucleotides (A, G, C, T). In some embodiments of the invention, this use is characterized in that the double-strand break is formed in a DNA molecule at a temperature of 37 oC to 65 oC. In preferred embodiments of the invention, this use is characterized in that the double-strand break is formed in a DNA molecule at a temperature of 37 oC to 55 oC.

Said problem is further solved by using a method for modifying a genomic DNA sequence of a unicellular or multicellular organism, comprising introducing into at least one cell of this organism an effective amount of: a) either a protein comprising the amino acid sequence of SEQ ID NO: 1, or a nucleic acid encoding the protein, comprising the amino acid sequence of SEQ ID NO: 1, and b) either a guide RNA comprising a sequence that forms a duplex with the nucleotide sequence of an organism's genomic DNA region, which is directly adjacent to the nucleotide sequence 5'-NNNNGNA-3' and interacts with said protein following the formation of the duplex, or a DNA sequence encoding said guide RNA, wherein interaction of said protein with the guide RNA and the nucleotide sequence 5'-NNNNGNA-3' results in the formation of a double-strand break in the genomic DNA sequence immediately adjacent to the sequence 5'-NNNNGNA-3'.

A mixture of crRNA and tracer RNA (tracrRNA), which can form a complex with a target DNA region and CcCas9 protein, may be used as a guide RNA. In preferred embodiments of the invention, a hybrid RNA constructed based on crRNA and tracer RNA may be used as a guide RNA. Methods for constructing a hybrid guide RNA are known to those skilled (Hsu PD, et al., DNA targeting specificity of RNA-guided Cas9 nucleases. Nat Biotechnol. 2013 Sep;31(9):827-32).

The invention may be used both for in vitro cutting target DNA, and for modifying the genome of some living organism. The genome may be modified in a direct fashion, i.e. by cutting the genome at a corresponding site, as well as by inserting an exogenous DNA sequence via homologous repair.

Any region of double-strand or single-strand DNA from the genome of an organism other than that used for administration (or a composition of such regions among themselves and with other DNA fragments) may be used as an exogenous DNA sequence, wherein said region (or composition of regions) is intended to be integrated into the site of a double-strand break in target DNA, induced by CcCas9 nuclease. In some embodiments of the invention, a region of double-strand DNA from the genome of an organism used for the introduction of CcCas9 protein, but further modified by mutations (substitution of nucleotides), as well as by insertions or deletions of one or more nucleotides, may be used as an exogenous DNA sequence.

The technical result of the present invention is to increase the versatility of the available CRISPR-Cas9 systems to enable the use of Cas9 nuclease for cutting genomic or plasmid DNA in a larger number of specific sites and wider temperature ranges.

### Brief description of the drawings

Fig. 1. Scheme of CRISPR loci in *Clostridium celluloliticum*
Fig. 2. Determination of PAM by *in vitro* methods. Development of a system for cutting DNA limited to the sequence NNNNGNA.
Fig. 3. Checking of significance of individual PAM positions.
Fig. 4. Checking of protein activity in cutting of various DNA targets.
Fig. 5. Reactions of in vitro cutting of a DNA fragment of the human grin2b gene
Fig. 6. Study of temperature range of CcCas9 activity.
Fig. 7. Scheme of interaction between the guide RNA and a region of target DNA.
Fig. 8. Alignment of sequences of Cas9 proteins from organisms Staphylococcus aureus (SaCas9), Campylobacter jejuni (CjCas9), and CcCas9.

Non-conserved regions of sequences are underlined.

### Detailed disclosure of the invention

As used in the description of the present invention, the terms "includes" and "including" shall be interpreted to mean "includes, among other things". Said terms are not intended to be interpreted as "consists only of". Unless defined separately, the technical and scientific terms in this application have typical meanings generally accepted in the scientific and technical literature.

As used herein, the term "percent homology of two sequences" is equivalent to the term "percent identity of two sequences". The identity of sequences is determined based on a reference sequence. Algorithms for sequence analysis are known in the art, such as BLAST described in Altschul et al., J. Mol. Biol., 215, pp. 403-10 (1990). For the purposes of the present invention, to determine the level of identity and similarity between nucleotide sequences and amino acid sequences, the comparison of nucleotide and amino acid sequences may be used, which is performed by the BLAST software package provided by the National Center for Biotechnology Information (http://www.ncbi.nlm.nih.gov/blast) using gapped alignment with standard parameters. Percent identity of two sequences is determined by the number of positions of identical amino acids in these two sequences, taking into account the number of gaps and the length of each gap to be entered for optimal comparison of the two sequences by alignment Percent identity is equal to the number of identical amino acids at given positions taking account of sequence alignment divided by the total number of positions and multiplied by 100.

The term "specifically hybridizes" refers to the association between two single-strand nucleic acid molecules or sufficiently complementary sequences, which permits such hybridization under pre-determined conditions typically used in the art.

The phrase "a double-strand break located **immediately** before the nucleotide PAM sequence" means that a double-strand break in a target DNA sequence will be made at a distance of 0 to 25 nucleotides before the nucleotide PAM sequence.

A protein comprising a specific amino acid sequence is intended to refer to a protein having an amino acid sequence composed of said amino acid sequence and possibly other sequences linked by peptide bonds to said amino acid sequence. An example of other sequences may be a nuclear localization signal (NLS), or other sequences that provide increased functionality for said amino acid sequence.

An exogenous DNA sequence introduced simultaneously with a guide RNA is intended to refer to a DNA sequence prepared specifically for the specific modification of a double-strand target DNA at the site of break determined by the specificity of the guide RNA. Such a modification may be, for example, an insertion or deletion of certain nucleotides at the site of a break in target DNA. The exogenous DNA may be either a DNA region from a different organism or a DNA region from the same organism as that of target DNA.

An effective amount of protein and RNA introduced into a cell is intended to refer to such an amount of protein and RNA that, when introduced into said cell, will be able to form a functional complex, i.e. a complex that will specifically bind to target DNA and produce therein a double-strand break at the site determined by the guide RNA and PAM sequence on DNA. The efficiency of this process may be assessed by analyzing target DNA isolated from said cell using conventional techniques known to those skilled.

A protein and RNA may be delivered to a cell by various techniques. For example, a protein may be delivered as a DNA plasmid that encodes a gene of this protein, as an mRNA for translation of this protein in cell cytoplasm, or as a ribonucleoprotein complex that includes this protein and a guide RNA. The delivery may be performed by various techniques known to those skilled.

The nucleic acid encoding system's components may be introduced into a cell directly or indirectly as follows: by way of transfection or transformation of cells by methods known to those skilled, by way of the use of a recombinant virus, by way of manipulations on the cell, such as DNA microinjection, etc.

A ribonucleic complex consisting of a nuclease and guide RNAs and exogenous DNA (if necessary) may be delivered by way of transfecting the complexes into a cell or by way of mechanically introducing the complex into a cell, for example, by way of microinjection.

A nucleic acid molecule encoding the protein to be introduced into a cell may be integrated into the chromosome or may be extrachromosomally replicating DNA. In some embodiments, to ensure effective expression of the protein gene with DNA introduced into a cell, it is necessary to modify the sequence of said DNA in accordance with the cell type in order to optimize the codons for expression, which is due to unequal frequencies of occurrence of synonymous codons in the coding regions of the genome of various organisms. Codon optimization is necessary to increase expression in animal, plant, fungal, or microorganism cells.

For a protein that has a sequence that is at least 95% identical to the amino acid sequence of SEQ ID NO: 1 to function in a eukaryotic cell, it is necessary for this protein to end up in the nucleus of this cell. Therefore, in some embodiments of the invention, a protein having a sequence that is at least 95% identical to the amino acid sequence of SEQ ID NO: 1 and which is further modified at one or both ends by the addition of one or more nuclear localization signals is used to form double-strand breaks in target DNA. For example, a nuclear localization signal from the SV40 virus may be used. To provide efficient delivery to the nucleus, the nuclear localization signal may be separated from the main protein sequence by a spacer sequence, for example, described in Shen B, et al. "Generation of gene-modified mice via Cas9/RNA- mediated gene targeting", Cell Res. 2013 May;23(5):720-3. Further, in other embodiments, a different nuclear localization signal or an alternative method for delivering said protein into the cell nucleus may be used.

The present invention encompasses the use of a protein from the organism of Clostridium cellulolyticum (*C. cellulolyticum*), which is homologous to the previously characterized Cas9 proteins, to introduce double-strand breaks into DNA molecules at strictly specified positions.

In metabolic engineering, editing the genome of *C. cellulolyticum* is a difficult task due to the lack of efficient editing tools. Methods for targeted genome editing, such as recombineering, group II intron retrotransposition, and allele exchange have a number of significant limitations. For example, the procedure of recombination-dependent allele exchange is rather time-consuming and has low efficiency. (Heap J. T. et al. Integration of DNA into bacterial chromosomes from plasmids without a counter-selection marker //Nucleic acids research. - 2012. - Vol. 40. - Issue 8. - pp. e59-e59). Insertion of long DNA fragments, such as metabolic pathway transfer, is difficult with current genome engineering tools, which require existing recombination sites and/or recombinases (Esvelt K. M., Wang H. H. Genome-scale engineering for systems and synthetic biology //Molecular systems biology. - 2013. - Vol. 9. - Issue 1. - p. 641). A simple and efficient method is needed for successful genome manipulations and production of mutants with pre-determined properties.

The use of CRISPR nucleases to introduce targeted modifications to the genome has a number of advantages. First, the specificity of the system's activity is determined by a crRNA sequence, which allows for the use of one type of nuclease for all target loci. Secondly, the technique enables the delivery of several guide RNAs complementary to different gene targets into a cell at once, thereby making it possible to simultaneously modify several genes at once.

Furthermore, the use of the native CRISPR-Cas9 system from the bacterium *C. cellulolyticum* will make the system for editing the genome of this organism more facile and efficient, as the procedure will not require the introduction of foreign genes into cells, maintenance and expression thereof. Instead, it is possible to develop a procedure for introducing guide RNAs, which are directed to target genes, into a bacterium, by means of which the host intracellular CRISPR-Cas9 system will be able to recognize the corresponding DNA targets of the biotechnologically significant bacterium and introduce therein double-strand breaks.

For the biochemical characterization of Cas9 protein from *C. cellulolyticum H10*, the CRISPR locus encoding the main system components (CcCas9, cas1, cas2 protein genes, as well as CRISPR cassette and guide RNAs) was cloned into the single copy bacterial vector pACYC184. The effector ribonucleic complex consisting of Cas9 and a crRNA/tracrRNA (tracer RNA) duplex requires the presence of PAM (protospacer adjusted motif) on a DNA target for recognition and subsequent hydrolysis of DNA, in addition to crRNA spacer-protospacer complementarity. (Mojica F. J. M. et al. Short motif sequences determine the targets of the prokaryotic CRISPR defence system //Microbiology. - 2009. - Vol. 155. - Issue 3. - pp. 733-740). PAM is a strictly defined sequence of several nucleotides located in type II systems adjacent to or several nucleotides away from the 3' end of the protospacer on an off-target chain. In the absence of PAM, the hydrolysis of DNA bonds with the formation of a double-strand break does not take place. The need for the presence of a PAM sequence on a target increases recognition specificity, but at the same time imposes restraints on the selection of target DNA regions for introducing a break.

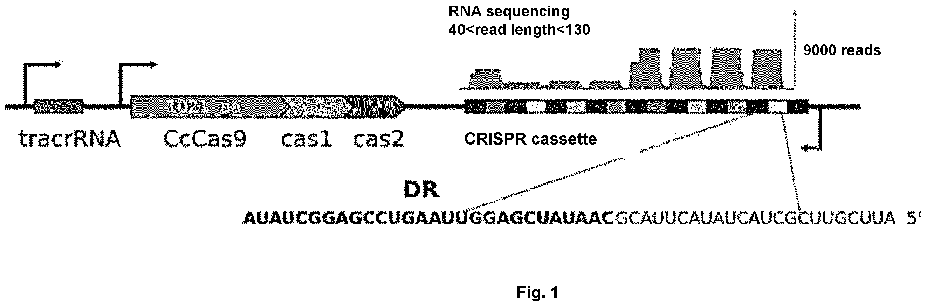

To determine the sequences of the guide RNA of the CRISPR-Cas9 system, RNA sequencing of *E.coli* DH5alpha bacteria bearing the generated pACYC184_CcCas9 construct was performed. The sequencing showed that the system's CRISPR cassette was actively transcribed, as was the tracer RNA **(****Fig. 1****).** Analysis of the crRNA and tracrRNA sequence allowed to contemplate that they may possibly form secondary structures recognized by CcCas9 nuclease.

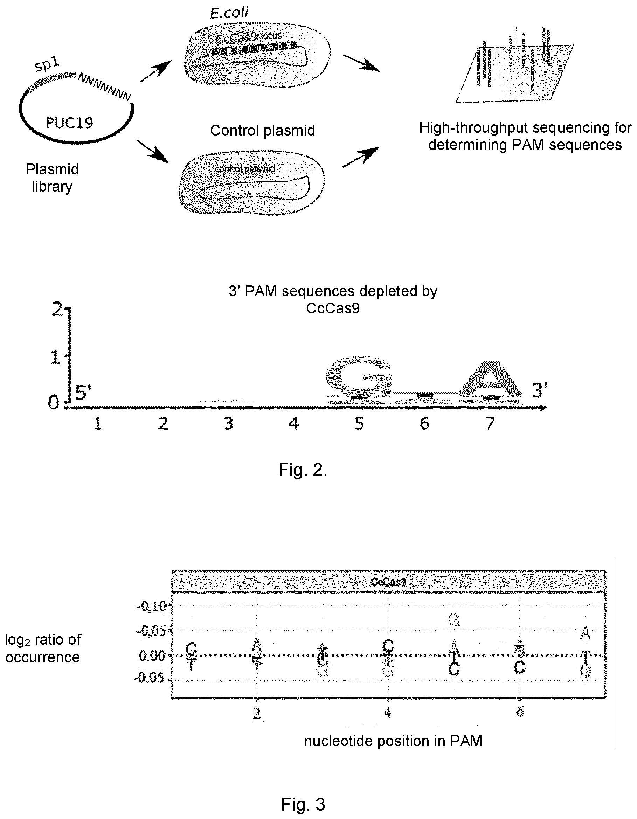

Further, the authors determined the PAM sequence of CcCas9 protein using bacterial PAM screening. In order to determine the PAM sequence of CcCas9 protein, *E.coli* DH5alpha cells bearing the pACYC184_CcCas9 plasmid were transformed by a plasmid library containing the spacer sequence 5'- TAAAAAATAAGCAAGCGATGATATGAATGC-3' of CRISPR cassette of CcCas9 system flanked by a random seven-letter sequence from the 5' or 3' end. Plasmids bearing a sequence corresponding to the PAM sequence of CcCas9 system were subjected to degradation under the action of functional CRISPR-Cas system, whereas the remaining library plasmids were effectively transformed into cells, conferring them resistance to the antibiotic ampicillin. After transformation and incubation of the cells on plates containing the antibiotic, the colonies were washed off the agar surface, and DNA was extracted therefrom using the Qiagen Plasmid Purification Midi kit. Regions containing the randomized PAM sequence were amplified by PCR from the isolated pool of plasmids, and were then subjected to high-throughput sequencing on the Illumina platform. The resulting reads were analyzed by comparing the efficiency of transformation of plasmids with unique PAMs included in the library into cells bearing pACYC184_CcCas9 or into control cells bearing the empty vector pacyc184. The results were analyzed using bioinformatics methods. As a result, it was possible to identify the PAM of the CcCas9 system, which is the two-letter sequence NNNNGNA (Fig. 2).

Next, the PAM sequence was further determined by reproducing the cutting reaction *in vitro.* To determine the PAM sequence of CcCas9 protein, *in vitro* cutting of double-strand PAM libraries was used. To this end, it was necessary to obtain all the components of the CcCas9 effector complex as follows: guide RNAs and a nuclease in a recombinant form. Determination of the guide RNA sequence by RNA sequencing made it possible to synthesize crRNA and tracrRNA molecules in vitro. The synthesis was carried out using the NEB HiScribe T7 RNA synthesis kit. The double-strand DNA libraries were 374 bp fragments comprising a protospacer sequence flanked by randomized seven nucleotides (5' NNNNNNN 3') from the 3' end:

5'cccggggtaccacggagagatggtggaaatcatctttctcgtgggcatccttgatggccacctcgtcggaa
gtgcccacgaggatgacagcaatgccaatgctgggggggctcttctgagaacgagctctgctgcctgacacggcca
ggacggccaacaccaaccagaacttgggagaacagcactccgctctgggcttcatcttcaactcgtcgactccctgc
aaacacaaagaaagagcatgttaaaataggatctacatcacgtaacctgtcttagaagaggctagatactgcaattc
aaggaccttatctcctttcattgagcacNNNNNNNaactccatctaccagcctactctcttatctctggtatt -3'

To cut this target, guide RNAs of the following sequence were used:
tracrRNA: 
5'AUUAUGGCAUAUCGGAGCCUGAAUUGUUGCUAUAAUAAGGUGCUGGG
UUUAGCCCAGACCGCCAAGUUAACCCCGGCAUUUAUUGCUGGGUAUCUUGU
UUU
and crRNA:
   5'**uaucuccuuucauugagcac**GUUAUAGCUCCAAUUCAGGCUCCGAUAU

Bold indicates the crRNA sequence complementary to the protospacer (target DNA sequence).

To obtain a recombinant CcCas9 protein, a gene thereof was cloned into the plasmid pET21a. E. coli Rosetta cells were transformed by the resulting plasmid CcCas9_pET21a. The cells bearing the plasmid were grown to an optical density of OD_600=0.6, then the expression of CcCas9 gene was induced by adding IPTG to a concentration of 1mM. The cells were incubated for 4 hours at 25 °C, after which they were lyzed. The recombinant protein was purified in two stages as follows: by affinity chromatography (NiNTA) and by protein size-exclusion on a Superdex 200 column. The resulting protein was concentrated using Amicon 30 kDa filters. Thereafter, the protein was frozen at minus 80°C and used for *in vitro* reactions.

The *in vitro* reaction of cutting linear PAM libraries was performed under the following conditions:
1xCutSmart buffer
400 nM CcSas9
100 nM DNA library
2 µM crRNA
2 µM tracrRNA

The total reaction volume was 20 µl.

*Clostridium cellulolyticum H10* is common in compost piles and has an optimal division temperature of 45°C, and, accordingly, the reactions were carried out at this temperature for 30 minutes.

The cutting resulted in the breaking-up of a portion of the library fragments into two portions having a length of about 50 base pairs (bp) and 324 bp. As a control sample, reactions without crRNA added, an essential component of the Cas effector complex, were used.

The reaction products were applied onto 1.5% agarose gel and subjected to electrophoresis. Uncut DNA fragments with a length of 374 bp were extracted from the gel and prepared for high-throughput sequencing using the NEB NextUltra II kit. The samples were sequenced on the Illumina platform and the sequences were then analyzed using bioinformatics methods where the difference in the representation of nucleotides at individual positions of PAM (NNNNNNN) was determined as compared to the control sample (Fig. 3).

As a result, the authors were able to determine the PAM sequence of CcCas9 by *in vitro* methods: NNNNGNA, which completely repeated the result obtained in experiments with bacteria.

Next, the significance of individual positions of the PAM sequence was checked. To this end, in vitro reactions were performed in cutting a DNA fragment comprising a DNA target 5'-gtgctcaatgaaaggagata-3' flanked by the PAM sequence GAGAGTA:

5'cccggggtaccacggagagatggtggaaatcatctttctcgtgggcatccttgatggccacctcgtcggaa
gtgcccacgaggatgacagcaatgccaatgctggggggctcttctgagaacgagctctgctgcctgacacggcca
ggacggccaacaccaaccagaacttgggagaacagcactccgctctgggcttcatcttcaactcgtcgactccctgc
aaacacaaagaaagagcatgttaaaataggatctacatcacgtaacctgtcttagaagaggctagatactgcaattc
aaggaccttatctcctttcattgagcac GAGAGTA aactccatctaccagcctactctcttatctctggtatt 3'

The reaction was performed under the following conditions:
1xCutSmart buffer
400 nM CcSas9
20 nM DNA
2 µM crRNA
2 µM tracrRNA

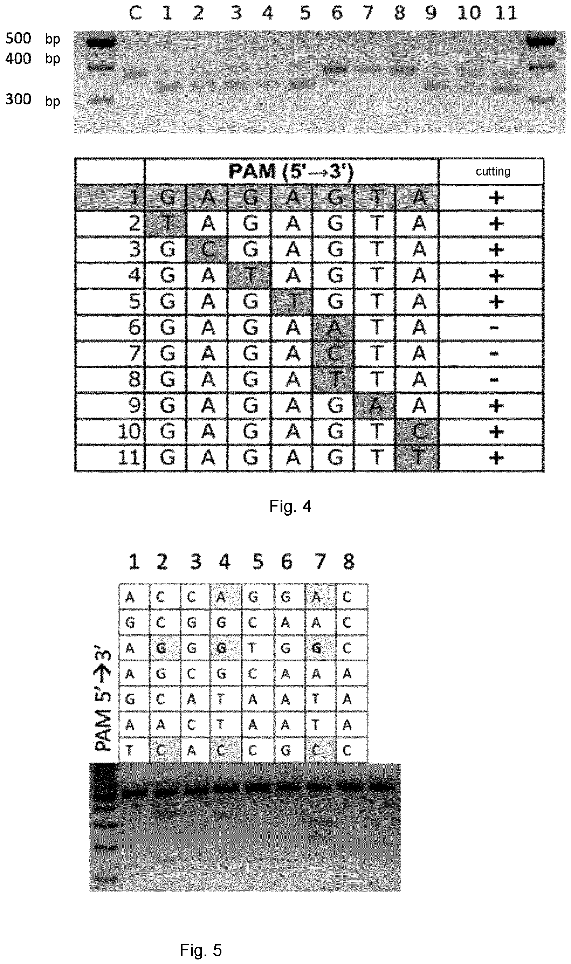

Incubation time was 30 minutes, reaction temperature was 45 °C. The experiment results confirmed the PAM sequence for CcCas9 as NNNNGNA -3'. The most conserved amino acid was G at position 5 (see fig. Fig. 4).

The following exemplary embodiments of the method are given for the purpose of disclosing the characteristics of the present invention and should not be construed as limiting in any way the scope of the invention.

### Example 1. Testing the activity of CcCas9 protein in the cutting of various DNA targets.

In order to check the ability of CcCas9 to recognize various DNA sequences flanked by the NNNNGNA 3' sequence, experiments were conducted on *in vitro* cutting of DNA targets from a human grin2b gene sequence (see Table 1 below).

**Table 1. DNA targets isolated from the human grin2b gene.**

| DNA target | PAM |
|---|---|
| ctacatcacgtaacctgtct | tagaAgA |
| gaacgagctctgctgcctga | cacgGcc |
| agaacgagctctgctgcctg | acacGgc |
| acggccaacaccaaccagaa | cttgGgA |
| tccgctctgggcttcatctt | caactcg |
| cgactccctgcaaacacaaa | gaaagag |
| atctacatcacgtaacctgt | cttaGaA |
| tatctcctttcattgagcac | caaaccc |

The in vitro DNA cutting reactions were performed under conditions similar to those of the above-described experiments. As a DNA target, a human grin2b gene fragment with a size of about 500 bp was used:

ttgtctctgcctgtagctgccaatgactatagcaatagcaccttttattgccttgttcaaggatttctgaggcttttga
aagtttcattttctctcattctgcagagcaaataccagagataagagagtaggctggtagatggagttgggtttggtgctc
aatgaaaggagataaggtccttgaattgcagtatctagcctcttctaagacaggttacgtgatgtagatcctattttaacat
gctctttctttgtgtttgcagggagtcgacgagttgaagatgaagcccagagcggagtgctgttctcccaagttctggttgg
tgttggccgtcctggccgtgtcaggcagcagagctcgttctcagaagagcccccccagcattggcattgctgtcatcctc
gtgggcacttccgacgaggtggccatcaaggatgcccacgagaaagatgatttccaccatctctccgtggtaccccg
gg

The experiment results show that CcCas9 in the complex with guide RNAs is able to recognize various DNA targets comprising the PAM sequence NNNNGNA (Fig. 5). In the case of some targets, CcCas9 is tolerant of substitutions at position 7 of the PAM sequence.

### Example 2. Temperature range of CcCas9 activity.

To determine the temperature range of the CcCas9 protein, experiments were conducted on in vitro cutting of a DNA target under different temperature conditions.

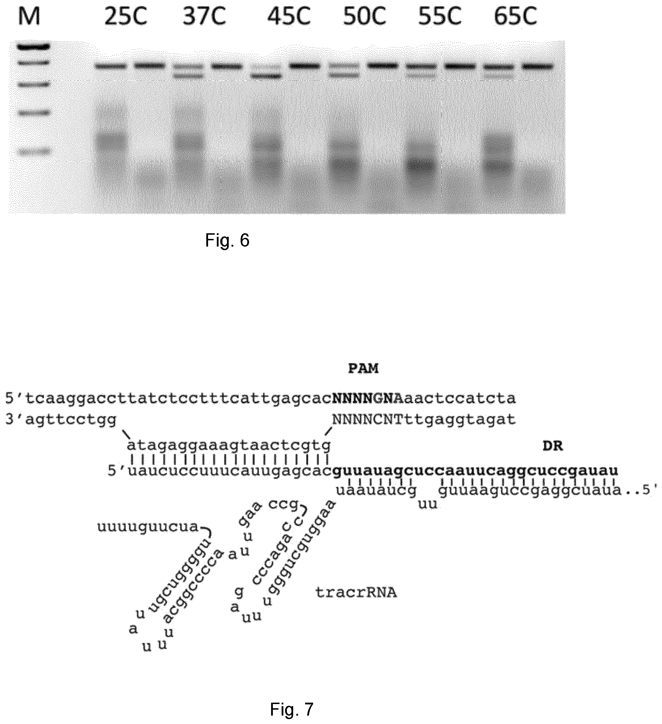

To this end, the target DNA flanked by the PAM sequence GAGAGTA was subjected to cutting by the CcCas9 effector complex with corresponding guide RNAs at different temperatures (Fig. 6).

The CcCas9 protein was found to have a wide temperature range of activity. The maximum nuclease activity is achieved at a temperature of 45 °C, whereas the protein is sufficiently active in the range of 37 °C to 55 °C. Hence, CcCas9 in the complex with guide RNAs is a novel tool for cutting (forming double-strand breaks) in a DNA molecule limited to the sequence 5'-NNNNGNA-3', with a temperature range of 37 °C to 55 °C. The scheme of the complex of target DNA with crRNA and tracer RNA (tracrRNA), which together form a guide RNA, is shown in Fig. 7.

### Example 3.

Cas9 proteins from closely related organisms belonging to *Clostridium.* Thus far, only one type II CRISPR Cas system has been found in Clostridium, which is Cas9 CRISPR Cas system from *Clostridium perfringens* (Maikova A, et al., New Insights Into Functions and Possible Applications of Clostridium difficile CRISPR-Cas System. Front Microbiol. 2018 Jul 31;9:1740).

The Cas9 protein from the bacterium *Clostridium perfringens* is identical to CcCas9 protein by 36% (degree of identity was calculated using the BLASTp software, default parameters). The Cas9 protein from *Staphylococcus aureus,* which is comparable in size, is identical to CcCas9 by 28% (BLASTp, default parameters).

Hence, the CcCas9 protein differs significantly in the amino acid sequence from other Cas9 proteins studied thus far, including those found in related organisms.

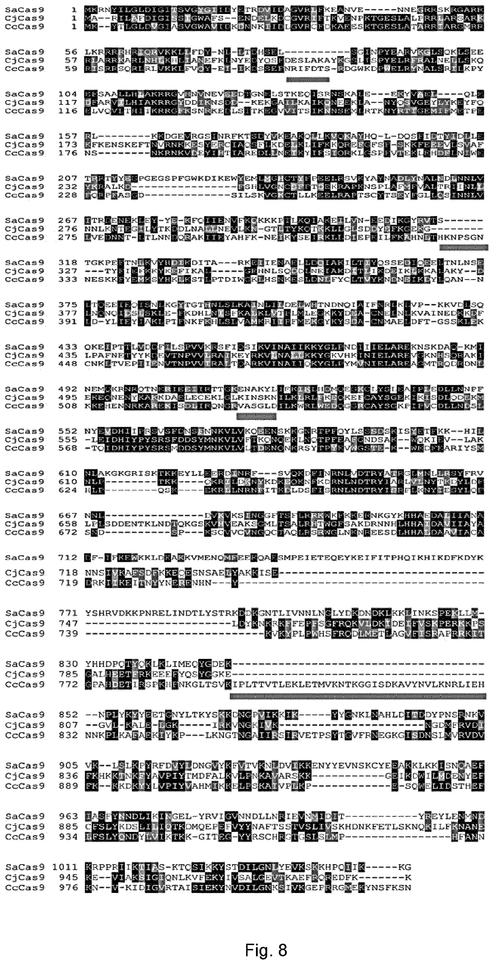

Those skilled in the art of genetic engineering will appreciate that CcCas9 protein sequence variant obtained and characterized by the Applicant may be modified without changing the function of the protein itself (for example, by directed mutagenesis of amino acid residues that do not directly influence the functional activity (Sambrook et al., Molecular Cloning: A Laboratory Manual, (1989), CSH Press, pp. 15.3-15.108)). In particular, those skilled will recognize that non-conserved amino acid residues may be modified, without affecting the residues that are responsible for protein functionality (determining protein function or structure). Examples of such modifications include the substitutions of non-conserved amino acid residues with homologous ones. Some of the regions containing non-conserved amino acid residues are shown in Figure 8. In some embodiments of the invention, it is possible to use a protein comprising an amino acid sequence that is at least 95% identical to the amino acid sequence of SEQ ID NO: 1 and differs from SEQ ID NO: 1 only in non-conserved amino acid residues, to form, in DNA molecule, a double-strand break located immediately before the nucleotide sequence 5'-NNNNGNA-3' in said DNA molecule. Homologous proteins may be obtained by mutagenesis (for example, site-directed or PCR-mediated mutagenesis) of corresponding nucleic acid molecules, followed by testing the encoded modified Cas9 protein for the preservation of its functions in accordance with the functional analyses described herein.

**Example 4.** The CcCas9 system described in the present invention, in combination with guide RNAs, may be used to modify the genomic DNA sequence of a multicellular organism, including a eukaryotic organism. For introducing the CcCas9 system in the complex with guide RNAs into the cells of this organism (into all cells or into a portion of cells), various approaches known to those skilled may be applied. For example, methods for delivering CRISPR-Cas9 systems to the cells of organisms have been disclosed in the sources (Liu C et al., Delivery strategies of the CRISPR-Cas9 gene-editing system for therapeutic applications. J Control Release. 2017 Nov 28;266:17-26; Lino CA et al., Delivering CRISPR: a review of the challenges and approaches. Drug Deliv. 2018 Nov;25(1):1234-1257), and in the sources further disclosed within these sources.

For effective expression of CcCas9 nuclease in eukaryotic cells, it will be desirable to optimize codons for the amino acid sequence of CcCas9 protein by methods known to those skilled (for example, IDT codon optimization tool).

For the effective activity of CcCas9 nuclease in eukaryotic cells, it is necessary to import the protein into the nucleus of a eukaryotic cell. This may be done by way of using a nuclear localization signal from SV40 T-antigen (Lanford et al., Cell, 1986, 46: 575-582) linked to CcCas9 sequence via a spacer sequence described in Shen B, et al. "Generation of gene-modified mice via Cas9/RNA- mediated gene targeting", Cell Res. 2013 May;23(5):720-3, or without the spacer sequence. Thus, the complete amino acid sequence of nuclease to be transported inside the nucleus of a eukaryotic cell will be the following sequence: MAPKKKRKVGIHGVPAA-CcCas9- KRPAATKKAGQAKKKK (hereinafter referred to as CcCas9 NLS). A protein with the above amino acid sequence may be delivered using at least two approaches.

Gene delivery is accomplished by creating a plasmid bearing the CcCas9 NLS gene under control of a promoter (for example, the CMV promoter) and a sequence encoding guide RNAs under control of the U6 promoter. As DNA targets, DNA sequences flanked by 5'-NNNNGNA-3' are used, for example, those of the human grin2b gene:

acggccaacaccaaccagaa

cgactccctgcaaacacaaa

Thus, the crRNA expression cassette looks as follows:

Gagggcctatttcccatgattccttcatatttgcatatacgatacaaggctgttagagagataattgga
attaatttgactgtaaacacaaagatattagtacaaaatacgtgacgtagaaagtaataatttcttgggtagtttg
cagttttaaaattatgttttaaaatggactatcatatgcttaccgtaacttgaaagtatttcgatttcttggctttatat
atcttgtggaaaggacgaaacacc-acggccaacaccaaccagaa-
GTTATAGCTCCAATTCAGGCTCCGATATttttt

Bold indicates the U6 promoter sequence, followed by the sequence required for target DNA recognition, while the direct repeat sequence is highlighted in capital letters.

The tracer RNA expression cassette looks as follows:

Gagggcctatttcccatgattccttcatatttgcatatacgatacaaggctgttagagagataattgga
attaatttgactgtaaacacaaagatattagtacaaaatacgtgacgtagaaagtaataatttcttgggtagtttg
cagttttaaaattatgttttaaaatggactatcatatgcttaccgtaacttgaaagtatttcgatttcttggctttatat
atcttgtggaaaggacgaaacaccATTATGGCATATCGGAGCCTGAATTGTTGCTATAAT
AAGGTGCTGGGTTTAGCCCAGACCGCCAAGTTAACCCCGGCATTTATTGCTGG
GGTATCTTTGtttt

Bold indicates the U6 promoter sequence, followed by the sequence encoding the tracer RNA. Plasmid DNA is purified and transfected into human HEK293 cells using Lipofectamine2000 reagent (Thermo Fisher Scientific). The cells are incubated for 72 hours, after which genomic DNA is extracted therefrom using genomic DNA purification columns (Thermo Fisher Scientific). The target DNA site is analyzed by sequencing on the Illumina platform in order to determine the number of insertions/deletions in DNA that take place in the target site due to a directed double-strand break followed by repair thereof.

Amplification of the target fragments is performed using primers flanking the presumptive site of break introduction, for example, for the above-mentioned grin2b gene sites:
5'-GACTATAGCAATAGCAC-3'
5'TCAACTCGTCGACTCCCTG-3'

After amplification, samples are prepared according to the Ultra II DNA Library Prep Kit for Illumina (NEB) reagent sample preparation protocol for high-throughput sequencing. Sequencing is then performed on the Illumina platform, 300 cycles, direct reading. The sequencing results are analyzed by bioinformatic methods. An insertion or deletion of several nucleotides in the target DNA sequence is taken as a cut detection.

Delivery as a ribonucleic complex is carried out by incubating recombinant CcCas9 NLS with guide RNAs in the CutSmart buffer (NEB). The recombinant protein is produced from bacterial producer cells by purifying the former by affinity chromatography (NiNTA, Qiagen) with size exclusion (Superdex 200).

The protein is mixed with RNAs in a ratio of 1:2:2 (CcCas9 NLS : crRNA :tracrRNA), the mixture is incubated for 10 minutes at room temperature, and then transfected into the cells.

Next, the DNA extracted therefrom is analyzed for insertions/deletions at the target DNA site (as described above).

The CcCas9 nuclease characterized in the present invention from the bacterium *Clostridium cellulolyticum* has a number of advantages relative to the previously characterized Cas9 proteins.

CcCas9 has a short, two-letter PAM, distinct from other known Cas nucleases, that is required for the system to function. Accrording to the authors, the short PAM GNA located 4 nucleotides away from the protospacer is sufficient for CcSas9. Further, G at position +5 is critical, whereas position +7 is less important, and *in vitro* hydrolysis was detected not only in the presence of A or T, but also in the presence of C at position +7, although with slightly lower efficiency.

The majority of Cas nucleases known thus far, which are capable of introducing double-strand breaks into DNA, have complex multi-letter PAM sequences, limiting the choice of sequences suitable for cutting. Among the Cas nucleases studied that recognize short PAMs, only CcCas9 is able to recognize sequences limited to GNA nucleotides.

The second advantage of CcCas9 is the small protein size (1030 a.a.r., which is 23 a.a.r. less as compared to that of SaCas9). To date, it is the only small-sized protein studied that has a two-letter PAM sequence.

The third advantage of the CcCas9 system is a wide temperature range of activity: the nuclease is active at temperatures of 37 oC to 65 oC with an optimum at 45 oC.

Although the invention has been described with reference to the disclosed embodiments, those skilled in the art will appreciate that the particular embodiments described in detail have been provided for the purpose of illustrating the present invention and are not be construed as in any way limiting the scope of the invention. It will be understood that various modifications may be made without departing from the spirit of the present invention.

## Claims

1. The use of a protein comprising the amino acid sequence of SEQ ID NO: 1, or comprising an amino acid sequence that is at least 95% identical to the amino acid sequence of SEQ ID NO: 1 and differs from SEQ ID NO: 1 only in non-conserved amino acid residues, to form a double-strand break in a DNA molecule, located immediately before the nucleotide sequence 5'-NNNNGNA-3'in said DNA molecule.

2. The use of the protein according to Claim 1, **characterized in that** the double-strand break is formed in the DNA molecule at a temperature of 37 °C to 65 °C.

3. The use of the protein according to Claim 1, wherein the protein comprises the amino acid sequence of SEQ ID NO: 1.

4. A method for generating a double-strand break in a genomic DNA sequence of a unicellular or multicellular organism directly adjacent to the sequence 5'-NNNNGNA-3', comprising the introduction into at least one cell of said organism of an effective amount of: a) a protein comprising the amino acid sequence of SEQ ID NO: 1, or a nucleic acid encoding the protein comprising the amino acid sequence of SEQ ID NO: 1, and b) a guide RNA comprising a sequence that forms a duplex with the nucleotide sequence of an organism's genomic DNA region, which is directly adjacent to the nucleotide sequence 5'-NNNNGNA-3' and interacts with said protein following the formation of the duplex, or a DNA sequence encoding said guide RNA,
wherein the interaction of said protein with said guide RNA and the nucleotide sequence 5'-NNNNGNA-3' results in the formation of a double-strand break in the genomic DNA sequence immediately adjacent to the sequence 5'-NNNNGNA-3'.

5. The method according to Claim 4 further comprising the introduction of an exogenous DNA sequence simultaneously with the guide RNA.
